# FASCICULE DE BREVET EUROPEEN

(11) **EP 2 933 251 B1**
(45) Date de publication et mention de la délivrance du brevet: **07.07.2021**
(21) Numéro de dépôt: 15163454.0
(22) Date de dépôt: 14.04.2015
(51) Int. Cl.: C07D 311/30, A61K 31/352, A61K 31/70, C07D 311/32, C07D 407/14, C07H 17/07, A61P 29/00

(54) **COMPOSITION PHARMACEUTIQUE UTILISABLE POUR LE TRAITEMENT D'UNE PATHOLOGIE DU PIED D'UN MAMMIFÈRE ONGULÉ**
PHARMAZEUTISCHE ZUSAMMENSETZUNG FÜR DIE BEHANDLUNG EINER FUSSKRANKHEIT EINES HUFTIERS
PHARMACEUTICAL COMPOSITION USABLE FOR THE TREATMENT OF A PATHOLOGY OF THE FOOT OF A HOOFED MAMMAL

(30) Priorité: 15.04.2014 FR 1453382
(43) Date de publication de la demande: 21.10.2015
(73) Titulaire: Weyts, Peter, 8690 Alveringem (BE); Bourgeois, Celine, 62170 Montcavrel (FR)
(72) Inventeur: Weyts, Peter M., 8690 Alveringem (BE)
(74) Mandataire: Ipside

(56) Documents cités:
- EP-A1- 1 970 059
- EP-A1- 2 208 498
- EP-A1- 2 353 596
- WO-A1-95/09635
- WO-A1-03/057210
- WO-A1-2005/058255
- WO-A1-2006/100468
- WO-A1-2012/012372
- WO-A1-2012/056113
- WO-A2-2007/011674
- WO-A2-2007/072535
- US-B1- 6 528 042
- GHOSH, MANIK ET AL: "GC-MS studies on the bark extracts of Litsea polyantha JUSS", MIDDLE EAST JOURNAL OF SCIENTIFIC RESEARCH, vol. 5, no. 6, 2010, pages 441-444, XP002730600,
- MIDDLETON E ET AL: "Effects of flavonoids on immune and inflammatory cell functions", BIOCHEMICAL PHARMACOLOGY, ELSEVIER, US, vol. 43, no. 6, 17 mars 1992 (1992-03-17), pages 1167-1179, XP025542458, ISSN: 0006-2952, DOI: 10.1016/0006-2952(92)90489-6 [extrait le 1992-03-17]
- MIDDLETON E ET AL: "THE EFFECTS OF PLANT FLAVONOIDS ON MAMMALIAN CELLS: IMPLICATIONS FOR INFLAMMATION, HEART DISEASE, AND CANCER", PHARMACOLOGICAL REVIEWS, AMERICAN SOCIETY FOR PHARMACOLOGY AND EXPERIMENTAL THERAPEUTICS, US, vol. 52, no. 4, 2000, pages 673-751, XP008047405, ISSN: 0031-6997

## Description

### DOMAINE TECHNIQUE

La présente invention concerne une composition pharmaceutique utilisable pour le traitement d'une pathologie du pied d'un mammifère ongulé.

### ART ANTERIEUR

Les mammifères ongulés comme les chevaux souffrent de nombreuses pathologies du pied.

Ainsi, la fourbure est une congestion inflammatoire aiguë du pied. Elle entraîne un arrêt circulatoire localisé avec ischémie des tissus. Le sang qui se loge dans la paroi de la fourchette du pied du cheval engendre le basculement de la troisième phalange vers l'avant : les chairs sont alors comprimées contre la paroi du sabot. Le traitement existant est une mise au repos, l'administration d'anti-inflammatoires et la pose d'une ferrure orthopédique.

S'agissant de la fourbure, le document WO 2006/100468 A1 indique que la N-acétylcystéine permet de réduire la contraction des vaisseaux sanguins du sabot du cheval. Il indique également que cet effet est plus marqué lorsque l'on combine la N-acétylcystéine avec de la génistine, un flavonoïde extrait du soja.

Le document WO 2007/011674 A2 indique lui, qu'une flavone polyméthoxylée combinée avec la catéchine peuvent être utilisées pour traiter la fourbure.

La bleime est une contusion de la sole du pied du cheval qui peut évoluer en hématome ou en abcès. Les bleimes peuvent être provoquées par des chocs sur une sole trop plate, par la présence d'un corps étranger ou par une mauvaise ferrure. Les bleimes peuvent entraîner une boiterie ou une simple baisse des performances. Le traitement repose sur l'extraction de l'éventuel corps étranger, le repos, l'administration d'anti-inflammatoires et l'application de pansements.

Par ailleurs, les chevaux de jumping ou d'endurance, par exemple, souffrent souvent d'inconfort provoqué par un échauffement du pied. En effet, les pieds subissent sans cesse de multiples micro-chocs qui engendrent l'augmentation de l'afflux sanguin ; il en résulte un échauffement du pied et un œdème. Les performances et la locomotion du cheval sont affectées par cet échauffement et les douleurs qu'il engendre. De plus, cet échauffement évolue parfois en bleime.

Des compositions pharmaceutiques contenant de la diosmine et de l'hespéridine sont connues. Ainsi, le document WO 95/09635 A1 décrit des compositions pharmaceutiques contenant de la diosmine et de l'hespéridine pour le traitement de l'herpès. Les documents EP 2 353 596 A1 et EP 2 208 498 A1 décrivent des compositions pharmaceutiques contenant notamment de la diosmine et de l'hespéridine pour le traitement de l'insuffisance veineuse chronique. Le document US 6 528 042 B1 décrit une composition contenant de la diosmine et de l'hespéridine qui présente des propriétés de cytoprotection.

Le document EP 1 970 059 A1 décrit une composition pharmaceutique pouvant être utilisée pour le traitement de la tendinite chez le cheval et comprenant de la diosmine.

Le document WO 2006/100468 A1 décrit l'art antérieur le plus proche. Ce document décrit un traitement de la fourbure du cheval laquelle peut provenir d'un désordre dans le système digestif de l'animal causé par la fermentation d'hydrates de carbone dans l'épigastre. Ce document indique qu'un antioxydant permet *in vitro* de réduire la contraction des vaisseaux sanguins du sabot due aux amines présentes dans le sang et provenant de l'ingestion d'hydrates de carbone. Cet effet est plus marqué lorsque l'on ajoute de la génistine, une isoflavone pouvant provenir du soja, notamment. D'autres flavonoïdes (la génistéine, la daïdzine, la daidzéine, la glycitine et la glycitéine) sont cités sans fournir de résultats expérimentaux. Le document cite l'hespéridine seule, sans combinaison avec la diosmine. Par ailleurs, le document n'envisage pas l'utilisation de deux composés flavonoïdes en combinaison pour le traitement de la fourbure.

### OBJECTIFS DE L'INVENTION

Un but de la présente invention est de proposer une composition pharmaceutique utilisable pour le traitement d'au moins une des pathologies précitées affectant le pied d'un mammifère ongulé, en particulier d'un équidé, et plus particulièrement un cheval.

Un autre but de la présente invention est de proposer une composition pharmaceutique qui permet de soulager l'inconfort ressenti par un mammifère onglé, notamment un cheval et notamment un cheval de jumping et/ou d'endurance.

Un autre but de la présente invention est de proposer une composition pharmaceutique qui permet de faire baisser la température du pied d'un mammifère ongulé, en particulier d'un équidé et plus particulièrement d'un cheval.

Un autre but de la présente invention est de proposer une solution à la baisse des performances des chevaux, notamment des chevaux des chevaux de sport et de courses.

### RESUME DE L'INVENTION

La présente invention propose une composition pharmaceutique, qui de manière caractéristique contient, en tant qu'ingrédients actifs, la diosmine et l'hespéridine pour son utilisation dans le traitement d'une pathologie du pied du cheval choisie parmi l'œdème du pied, la fourbure, la bleime, l'inconfort du pied, en particulier, l'inconfort causé par une augmentation de la température du pied/sabot (échauffement), les altérations de la circulation sanguine dans le pied, plus précisément au niveau du sabot et/ou des tissus mous présents à proximité du sabot et les douleurs de ferrure.

Sans être liés par l'explication suivante, les Demandeurs considèrent que les composés et la composition selon l'invention agissent sur la dilatation des veinules et artérioles alimentant le pied et en particulier le sabot. Certains médiateurs (comme l'histamine, par exemple) provenant de la circulation générale ou produits par l'organisme de l'animal sous l'effet des chocs, par exemple, agissent sur les muscles péri veineux et/ou péri capillaires augmentant ainsi leur diamètre. La circulation sanguine étant augmentée dans les veines et capillaires, le sang empreinte les anastomoses présentes dans le pied et engendre de ce fait un œdème des tissus du pied, au niveau du sabot notamment, associé à une compression interne des tissus qui engendre une élévation de température. La composition selon l'invention permet grâce à la vasoconstriction des veines et/ou veinules et/ou capillaires de rétablir la circulation sanguine normale dans le pied/sabot et de réduire ainsi l'œdème, réduire la pression interne et faire ainsi baisser la température du pied.

### DESCRIPTION DETAILLEE

L'excipient n'est pas limité selon l'invention. Il peut, à titre d'exemple, être choisi parmi la maltodextrine, la gomme xanthane, l'eau, l'agar-agar, la gélatine, le saccharose, le lactose, l'amidon, l'amidon modifié et le propylène glycol et les mélanges d'au moins deux des composants précités.

La composition peut être adaptée pour être administrée par voie orale.

Selon un mode de réalisation, elle contient en tant qu'ingrédient actif un mélange de diosmine et d'hespéridine contenant en masse une quantité de diosmine sensiblement égale ou supérieure 80% et sensiblement égale ou inférieure 95% et en particulier sensiblement égale à 90%.

La composition selon l'invention peut contenir une quantité d'ingrédient(s) actif(s) sensiblement égale ou supérieure à 4g et sensiblement égale ou inférieure à 8 g et de préférence sensiblement égale à 6,25g.

Elle peut être est contenue dans une seringue.

La présente invention concerne également une seringue contenant la composition selon l'invention.

La présente invention concerne également un complément alimentaire à usage vétérinaire, qui contient une composition pharmaceutique selon l'invention.

Le mode d'administration de la composition selon l'invention n'est pas limité. Elle peut être administrée par voie orale, intradermique, sous-cutanée, intramusculaire, mucosale (voie rectale, intra nasale, sublinguale). La composition peut donc, par exemple, se présenter sous la forme d'un solide, d'une poudre, par exemple, d'un liquide, d'une suspension ou d'un colloïde. Elle peut également se présenter sous la forme d'un comprimé, d'un suppositoire, d'une gélule, d'une pommade, d'une crème ou d'un patch cutané.

Avantageusement, la composition selon l'invention est adaptée pour être administrée par voie orale. Elle se présente de manière avantageuse sous la forme d'un liquide ou d'un gel qui peut facilement être administré par voie orale à l'aide d'une seringue, par exemple.

La quantité d'ingrédient(s) actif(s) n'est pas limitée selon l'invention et l'Homme du Métier est à même de déterminer la quantité nécessaire pour obtenir un effet thérapeutique. Néanmoins, les Demandeurs ont constaté qu'une quantité d'ingrédient(s) actifs sensiblement égale ou supérieure à 4g et sensiblement égale ou inférieure à 8g et de préférence sensiblement égale à 6,25g s'avérait particulièrement efficace.

Selon la pathologie traitée, la dose journalière de composés selon l'invention est supérieure ou sensiblement égale 4g, avantageusement sensiblement égale à 6,25g et inférieure ou sensiblement égale à 13g.

La dose journalière par kilogramme est sensiblement égale ou supérieure à 0,01g/kg, avantageusement sensiblement égale à 0,016g/kg et inférieure ou sensiblement égale à 0,045g/kg. La dose journalière peut être sensiblement égale à 0,032g/kg.

Pour le traitement de la bleime et de la fourbure, la composition selon l'invention est administrée, par exemple en deux prises par jours pendant trois jours.

Pour le traitement de l'inconfort et des douleurs des suites de ferrure, la composition peut être administrée une fois par jour, avantageusement en une dose journalière telle que précitée.

Les Demandeurs ont également constaté que l'administration par voie orale de la composition de l'invention, à distance des repas s'avérait plus efficace.

### DEFINITIONS

Un « mammifère ongulé » désigne, au sens de la présente invention, tout mammifère dont les doigts sont recouverts d'un ou plusieurs sabots, tel que, par exemple, la chèvre, le cochon, l'hippopotame, la vache, le cheval, l'âne, le mulet, le zèbre, le rhinocéros, le tapir et l'éléphant.

Un « équidé » est défini au sens de la présente invention comme étant un mammifère appartenant à l'espèce des chevaux, des ânes, des zèbres et des mammifères issus des croisements entre deux de ces espèces.

Le terme « traitement » englobe au sens de la présente invention le traitement préventif et le traitement curatif. Ainsi, au sens de la présente invention, le terme « traitement » désigne notamment toute amélioration d'au moins un symptôme lié à une pathologie et également toute amélioration de l'état d'une région du corps dudit mammifère pouvant à plus ou moins long terme engendrer une pathologie. L'amélioration de l'inconfort et/ou la suppression ou la réduction de la douleur sont également considérées comme des traitements au sens de la présente invention.

Le terme « excipient » désigne toute substance inerte d'un point de vue thérapeutique et servant à la formulation de la forme galénique de la composition selon l'invention.

Les termes «pharmaceutique » et pharmaceuticalement acceptable » désignent tout produit qui ne génère pas d'effet secondaire supérieur aux avantages procurés par le traitement. A titre d'exemple, tout produit pouvant permettre au moins une administration par voie cutanée sans engendrer des effets secondaires indésirables d'une ampleur pouvant permettre l'arrêt du traitement est un produit pharmaceutique ou pharmaceuticalement acceptable selon la présente invention.

Les termes « à distance des repas », désignent une administration décalée dans le temps par rapport à la prise d'aliments, en particulier une administration décalée de deux heures, de trois heures, de quatre heures et de préférence de six heures de toute prise alimentaire.

Le terme « température du pied » désigne la température mesurée sur la ligne de la couronne de la boîte cornée.

### FIGURES

- la Fig. 1 représente la radiographie du pied d'un cheval atteint de fourbure avant le traitement avec la composition selon l'invention ;
- la Fig. 2 représente la radiographie du pied du même cheval que celui en référence à la Fig. 1, prise 10 jours après la première administration de l'exemple de composition selon l'invention décrit ci-après.

### PARTIE EXPERIMENTALE

### Préparation d'un exemple de composition

1g d'hespéridine sous forme de poudre est mélangé à 9 g de diosmine également sous forme de poudre. On prélève 6,25g du mélange précité en poudre et on les mélange à 25mL d'excipient gomme xanthane commercialisé par la société Fagron. L'excipient gomme de xanthane présente la composition suivante pour 100mL d'excipient: gomme xanthane (664mg), sodium saccharinate, sorbitol liquide, propylène glycol glycérol, méthyle parabène (150mg), propyl parabène (30mg), arôme de framboise, eau purifiée (100mL)

Les 25mL de gel ainsi formés sont introduits dans une seringue pour administration par voie orale chez le cheval. Cette seringue contient donc une dose administrable et procurant un effet thérapeutique.

### Traitement de la fourbure

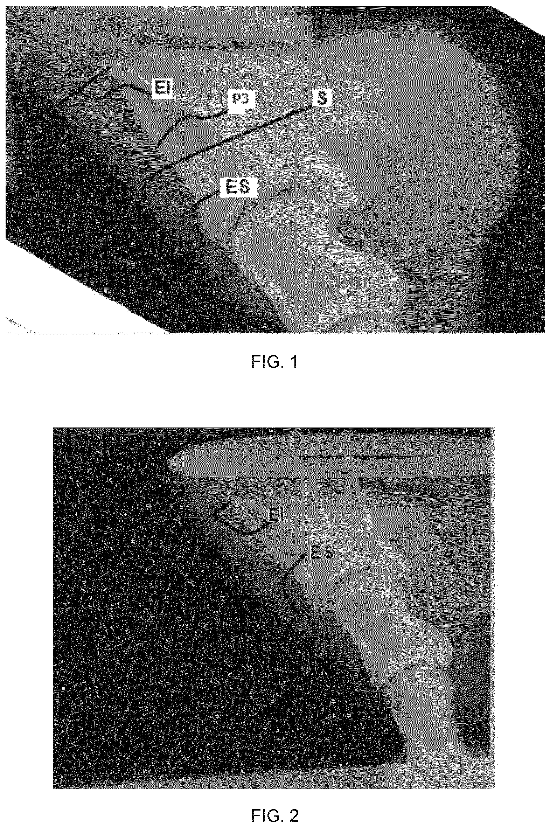

La Fig. 1 représente le pied d'un cheval atteint de fourbure. Comme cela est visible sur la Fig. 1, le pied du cheval comporte un sabot corné S qui recouvre l'extrémité d'une phalange P3. Des chairs alimentées par des vaisseaux sanguins se trouvent à l'arrière de la phalange. Sur la Fig. 1, on diagnostique facilement la fourbure par le fait que la phalange P3 bascule vers l'avant ; il s'ensuit que la phalange P3 se décolle du sabot S et la distance ES qui sépare le haut de la phalange P3 du bord longitudinal du sabot est donc plus petite que la distance EI séparant l'extrémité de la phalange P3 du bord longitudinal du sabot.

La composition de l'exemple précité a été administrée au cheval souffrant de fourbure et dont la Fig. 1 représente une radiographie du pied. 25mL de la composition décrite dans l'exemple précité ont été administrés par voie orale 2 fois par jours, à 12 heures d'intervalle pendant 8 jours. 3 heures séparaient l'administration d'une dose et une prise alimentaire. La Fig. 2 représente la radiographie du pied du même cheval prise après le traitement précité. Comme cela est visible sur la Fig. 2, on constate que la fourbure est résorbée, la distance ES étant sensiblement égale à la distance EI, il n'y a donc plus de trace de basculement de la phalange P3 vers l'avant.

Aucune récidive n'a été observée.

### Traitement de la bleime

25mL de la composition de l'exemple précité sont administrés par voie orale 2 fois par jour à 12 heures d'intervalle pendant 4 jours. La résorption rapide de l'hématome évite en général la formation d'un abcès signe d'une infection bactérienne. Aucune récidive n'a été observée.

### Traitement de l'inconfort du pied

L'inconfort du pied qui engendre bien souvent des problèmes de locomotion du cheval provient d'une augmentation de la température à l'intérieur de la boîte cornée. Cette augmentation de température est révélatrice d'une augmentation de la pression sanguine interne. La température idéale mesurée sur la ligne de la couronne de la boîte cornée est inférieure ou égale à 20°C. A l'effort, le pied s'échauffe et sa température peut atteindre 24°C.

Les Demandeurs ont constaté qu'une administration par voie orale de 25mL de la composition selon l'exemple précité permettait en 2 ou trois heures de diminuer la température du sabot mesurée sur la ligne de la couronne. Ainsi, si la température du sabot mesurée sur la ligne de la couronne dépasse 24°C, après administration de la composition selon l'exemple précité, on constate une diminution dans les 2, 3 heures de 3 à 4°C et une nette amélioration de la locomotion de l'animal. La composition selon l'exemple précité peut également être administrée à titre préventif. Ainsi, si l'on administre par voie orale une dose de 25mL de la composition selon l'exemple précité 4 heures avant l'effort, on n'observe aucune élévation de la température du sabot mesurée sur la ligne de la couronne. On veillera à ne pas dépasser une dose de 25mL par 24 heures dans le cas du traitement préventif de l'inconfort du pied.

### Traitement des douleurs après ferrure

La pose de nouveaux fers s'accompagne de douleurs causées probablement par le travail de taille du sabot du maréchal ferrant, les chocs durant la pose des fers et la présence des nouveaux fers. Ces douleurs durent quelques jours après la pose des fers et se diagnostiquent par une locomotion altérée de l'animal. Les Demandeurs ont constaté que l'administration d'une dose journalière de 25mL du gel de l'exemple de composition selon l'invention précité améliorait très rapidement la locomotion ce qui signifie que les douleurs dues à la nouvelle ferrure sont réduites voire supprimées. Une dose de 25mL de l'exemple de composition précité est administrée une fois par jour pendant trois jours pour traiter les douleurs et la locomotion altérée dues à la pose de nouveaux fers.

## Revendications

1. Composition pharmaceutique, contenant en tant qu'ingrédients actifs, la diosmine et l'hespéridine pour son utilisation dans le traitement d'une pathologie du pied du cheval choisie parmi l'œdème du pied, la fourbure, la bleime, l'inconfort du pied, en particulier, l'inconfort causé par une augmentation de la température du pied/sabot (échauffement), les altérations de la circulation sanguine dans le pied, plus précisément au niveau du sabot et/ou des tissus mous présents à proximité du sabot et les douleurs de ferrure.

2. Composition pharmaceutique selon la revendication précédente, pour son utilisation selon la revendication 1, **caractérisée en ce qu'**elle contient en tant qu'ingrédient actif un mélange de diosmine et d'hespéridine contenant en masse une quantité de diosmine sensiblement égale ou supérieure 80% et sensiblement égale ou inférieure 95% et en particulier sensiblement égale à 90%.

3. Composition pharmaceutique selon l'une quelconque des revendications précédentes, pour son utilisation selon la revendication 1, **caractérisée en ce qu'**elle contient une quantité d'ingrédient(s) actif(s) sensiblement égale ou supérieure à 4g et sensiblement égale ou inférieure à 8 g et de préférence sensiblement égale à 6,25g.

4. Composition pharmaceutique selon l'une quelconque des revendications précédentes, pour son utilisation selon la revendication 1, **caractérisée en ce qu'**elle est contenue dans une seringue adaptée à une administration par voie orale.

5. Complément alimentaire à usage vétérinaire, **caractérisé en ce qu'**il contient en tant qu'ingrédient actif un mélange de diosmine et d'hespéridine.

6. Complément alimentaire selon la revendication précédente, **caractérisé en ce que** ledit mélange de diosmine et d'hespéridine contient en masse une quantité de diosmine sensiblement égale ou supérieure 80% et sensiblement égale ou inférieure 95% et en particulier sensiblement égale à 90%.

7. Complément alimentaire selon l'une quelconque des revendications précédentes, **caractérisé en ce qu'**il contient une quantité d'ingrédient actif sensiblement égale ou supérieure à 4g et sensiblement égale ou inférieure à 8 g et de préférence sensiblement égale à 6,25g.

## Patentansprüche

1. Pharmazeutische Zusammensetzung, die als aktive Inhaltsstoffe Diosmin und Hesperidin für ihre Verwendung bei der Behandlung einer Erkrankung des Fußes des Pferds enthält, ausgewählt aus dem Fußödem, der Hufrehe, der Steingalle, den Fußbeschwerden, insbesondere den Beschwerden aufgrund einer Erhöhung der Temperatur des Fußes/Hufs (Erwärmung), den Beeinträchtigungen der Blutzirkulation im Fuß, genauer im Bereich des Hufs und/oder der Weichgewebe in der Nähe des Hufs und den Hufbeschlagschmerzen.

2. Pharmazeutische Zusammensetzung nach vorangehendem Anspruch für ihre Verwendung nach Anspruch 1, **dadurch gekennzeichnet, dass** sie als aktiven Inhaltsstoff ein Gemisch aus Diosmin und Hesperidin enthält, das in Masse eine Diosminmenge enthält, die etwa gleich oder über 80 % und etwa gleich oder unter 95 % und insbesondere etwa gleich 90 % ist.

3. Pharmazeutische Zusammensetzung nach einem der vorangehenden Ansprüche für ihre Verwendung nach Anspruch 1, **dadurch gekennzeichnet, dass** sie eine Menge aktiven/aktiver Inhaltsstoffs/Inhaltsstoffe von etwa gleich oder über 4 g und etwa gleich oder unter 8 g und vorzugsweise von etwa gleich 6,25 g enthält.

4. Pharmazeutische Zusammensetzung nach einem der vorangehenden Ansprüche für ihre Verwendung nach Anspruch 1, **dadurch gekennzeichnet, dass** sie in einer Spritze enthalten ist, die für eine orale Verabreichung geeignet ist.

5. Nahrungsergänzung für den tierärztlichen Gebrauch, **dadurch gekennzeichnet, dass** sie als aktiven Inhaltsstoff ein Gemisch aus Diosmin und Hesperidin enthält.

6. Nahrungsergänzung nach vorangehendem Anspruch, **dadurch gekennzeichnet, dass** das Gemisch aus Diosmin und Hesperidin in Masse eine Diosminmenge enthält, die etwa gleich oder über 80 % und etwa gleich oder unter 95 % und insbesondere etwa gleich 90 % ist.

7. Nahrungsergänzung nach einem der vorangehenden Ansprüche, **dadurch gekennzeichnet, dass** sie eine Menge aktiven Inhaltsstoffs von etwa gleich oder über 4 g und etwa gleich oder unter 8 g und vorzugsweise von etwa gleich 6,25 g enthält.

## Claims

1. A pharmaceutical composition, containing as active ingredients, diosmin and hesperidin, for use in the treatment of a pathology of the horse's foot selected from pedal oedema, laminitis, sand-crack, foot discomfort, in particular, the discomfort caused by an increase in the temperature of the foot/hoof (heating), the alterations in blood circulation in the foot, more specifically at the hoof and/or the soft tissues which are present in the vicinity of the hoof and the shoeing pains.

2. The pharmaceutical composition according to the preceding claim, for use according to claim 1, **characterised in that** it contains as active ingredient a mixture of diosmin and hesperidin containing by mass an amount of diosmin which is substantially equal to or greater than 80% and substantially equal to or less than 95% and in particular substantially equal to 90%.

3. The pharmaceutical composition according to any of the preceding claims, for use according to claim 1, **characterised in that** it contains an amount of active ingredients(s) which is substantially equal to or greater than 4 g and substantially equal to or less than 8 g and preferably substantially equal to 6.25 g.

4. The pharmaceutical composition according to any of the preceding claims, for use according to claim 1, **characterised in that** it is contained in a syringe suitable for oral administration.

5. A food supplement for veterinary use, **characterised in that** it contains as active ingredient a mixture of diosmin and hesperidin.

6. The food supplement according to the preceding claim, **characterised in that** said mixture of diosmin and hesperidin contains by mass an amount of diosmin which is substantially equal to or greater than 80% and substantially equal to or less than 95% and in particular substantially equal to 90%.

7. The food supplement according to any of the preceding claims, **characterised in that** it contains an amount of active ingredient which is substantially equal to or greater than 4 g and substantially equal to or less than 8 g and preferably substantially equal to 6.25 g.
